# EUROPEAN PATENT SPECIFICATION

(11) **EP 2 370 351 B1**
(45) Date of publication and mention of the grant of the patent: **09.03.2016**
(21) Application number: 09835778.3
(22) Date of filing: 22.12.2009
(51) Int. Cl.: C01B 11/02, A61C 5/00

(54) **COMPOSITION AND METHOD FOR REDUCING DEMINERALIZATION OF TEETH**
ZUSAMMENSETZUNG UND VERFAHREN ZUR VERRINGERUNG VON ZAHNENTMINERALISIERUNG
COMPOSITION ET PROCÉDÉ DE RÉDUCTION DE LA DÉMINÉRALISATION DENTAIRE

(30) Priority: 22.12.2008 US 140010 P
(43) Date of publication of application: 05.10.2011
(73) Proprietor: Micropure, INC., Scottsdale, AZ 85260 (US)
(72) Inventor: RATCLIFF, James, L., Scottsdale, Arizona 85255 (US); KIRSCH, Lee, E., Iowa City IA 52245 (US); DYKSTRA, Jessica, Ward, Gilbert Arizona 85297 (US); COOLEY, William, E., Wyoming OH 45215 (US); ARMITAGE, Gary, San Francisco CA 94116 (US); ASHLEY, Robert, Encinitas California 92024 (US); GARCIA, Esmeralda, Ann, Arizona 85032 (US)
(74) Representative: Lane, Cathal Michael
(86) International application number: PCT/US2009/069253
(87) International publication number: WO 2010/075419

(56) References cited:
- WO-A1-2007/077210
- US-A- 5 348 734
- US-A1- 2004 126 335
- US-B2- 6 582 682

## Description

### BACKGROUND OF THE INVENTION

### 1. Field of the Invention:

The present invention is directed to an oral care product and method and, more particularly to a composition and method for preventing caries.

### 2. Description of Related Art

For many years, oral care regimens have been used by consumers and dental professionals to safely and effectively prevent and treat a range of oral conditions or diseases, such as periodontitis and dental caries. Toothpastes, oral rinses, oral gels, and oral sprays are among the types of oral care products included in these regimens. A well-studied example of the utility of oral care products against human oral disease is the application of fluoride toothpaste to teeth and the oral cavity for the prevention of dental caries, which has shown significant effects in contributing to overall decline of caries prevalence in whole human populations. (2008 Cury) As a result of the proven anti-caries efficacy of oral care products containing fluoride, government and industry standards have been established to ensure the delivery of safe and effective over the counter, anti-caries drug products to the public. One such example of these standards is codified in the United States (US) Food and Drug Administration (FDA) anti-caries monograph, United States Code (USC) Title 21 Parts 310, 355, and 369.

### Dental Caries: Pathophysiology

Dental caries, or tooth decay, is a complex and widespread disease characterized by carious lesions that form on the tooth. The caries process initially occurs at the surface of the tooth where dental biofilm is present. Dental biofilm is composed of "an acquired enamel pellicle [a salivary protein pellicle] and dental plaque." (2008 Garcia-Godoy) The acquired enamel pellicle (AEP) serves as a structure that microbes use to initially attach to the tooth and subsequently, aerobic and anaerobic microbes colonize and coaggregate to form dental plaque. Taken together, as AEP and plaque develop, these structures mature into a dental biofilm. Cariogenic bacteria inhabit the dental biofilm and ferment dietary sugars (such as sucrose) into acid to create an acidic environment near the tooth. This localized acidic pH and shift back toward a less acidic, resting pH contribute to the demineralization and remineralization of the tooth, respectively. When the demineralization/remineralization process results in a net mineral loss in the tooth, the hard tissues of the tooth may dissolve and a caries lesion may develop. (2008 Garcia Godoy, 2004 Kidd, 2000 Lendennman, 2007 Islam)

Demineralization and remineralization (Demin/Remin) are dynamic events that occur regularly in the mineralized tissue of the teeth, specifically the enamel and dentin which maintain their structural integrity due to the abundance of mineralized carbonated hydroxyapatite (HAP) in these tissues [the hydroxyapatite in these tissues "can be approximately represented by the following formula: Ca10-x (Na)x (PO)6-y (CO3) z (OH)2-u (F)u"]. (2007 Islam) Under normal, physiological conditions, saliva and biofilm contain a supersaturation of calcium ions and phosphate (Pi) compared to the mineralized tooth, and as a result of this supersaturation, these oral fluids are able to mineralize the tooth with calcium and Pi. When an acidic pH results from the metabolic activity of cariogenic bacteria in dental biofilm, the biofilm fluid becomes undersaturated with calcium and Pi, compared with the tooth, which leads to the release of calcium and Pi from the tooth into the dental biofilm - this event is demineralization. The calcium and Pi lost, from the enamel, results from the dissolution of hydroxyapatite. As the pH returns to physiological (less acidic) pH, the supersaturated state of the oral fluid returns and biofilm and saliva are again able to mineralize the tooth with calcium and Pi, which helps restore some the mineral lost by the tooth during demineralization - this event is remineralization. (2008 Garcia-Godoy, 2008 Islam, 2009 Cury, 2008 Cury, 2007 Braly, 2004 Kidd)

### Dental Caries: Factors in Progression

Factors that contribute to deterioration of healthy tooth enamel and dental caries formation include but are not limited to: "cariogenic and noncariogenic bacteria, salivary components (proteins, enzymes, calcium, phosphate, fluoride), and dietary sources of fermentable carbohydrates (sucrose, glucose)." It is well known in the art that the gram positive, anaerobic bacteria *Streptococcus mutans* is the primary cariogenic bacteria due to its aciduric nature, presence in dental biofilm, and ability to readily ferment sugars (like sucrose) into acid which helps create the acidic environment necessary for demineralization. Other known cariogenic bacteria include *Streptococcus sobrinus, Lactobacillus* species (spp), and *Actinomyces* spp. (2008 Garcia Godoy, 2007 Islam)

Normal human saliva may contain various biological and chemical components (including calcium, Pi, fluoride, lactoferrin, lysozymes, proteases, and glycoproteins). A 2001 literature review of caries studies assessing salivary factors and caries risk states that "chronically low salivary flow rate (for example <0.8 1.0 ml/min stimulated whole saliva)" is "the strongest indicator of an increased risk for caries prevalence or incidence." (2001 Leone) This assertion is based on twenty-one studies that observed a specific association in increased caries risk and individuals with medical conditions (like Sjögren's syndrome) that affect the normal function of the salivary gland. It is difficult to establish a significant relationship between the concentration of a specific salivary component and caries risk because dental caries is caused by many different factors and the composition of saliva may vary from person to person. (1995 Edgar, 2001 Leone, 2008 Garcia Godoy)

A concept entitled the 'Caries Balance' states two categories of factors to assess risk for caries. One category is known as pathological factors, which are factors that contribute to caries progression. Pathological factors include the presence of acidogenic, cariogenic bacteria, regular consumption of fermentable sugars, and a reduction in salivary flow. A second category of factors are known as protective factors, which are factors that provide helpful anti-caries effects to the tooth. Protective factors include the presence of normal salivary flow and salivary components, fluoride and other minerals that enhance tooth remineralization, and antibacterial agents capable of challenging cariogenic bacteria particularly in subjects at high risk for caries. (2006 Featherstone, 2008 Garcia Godoy)

### Dental Caries: Fluoride as an Anti-Caries Oral Care Preventative and Treatment

Fluoride is a widely used highly effective agent. The use of fluoride does not eliminate the pathological factors of caries (such as dental biofilm or cariogenic bacteria). Rather, fluoride disrupts the caries process at the site of occurrence through the protective physiochemical effects it confers to the tooth throughout the demineralization and remineralization "processes taking place at the interface between the tooth surface and the oral fluids". (2008 Cury, 2002 Aoba) During demineralization (where pH is > 4.5) when hydroxyapatite is dissolved to release calcium and Pi, fluoride is able to recover some of this calcium and Pi by forming fluorapatite, which is not as acid soluble as hydroxyapatite. In this way, fluoride reduces demineralization by helping to maintain the mineralized state of the enamel against acid induced mineral loss. (2009 Cury, 2008 Cury, 2008 Islam, 2000 Robinson) Fluoride also enhances remineralization because when the pH in the localized oral environment is restored to > 5.5, the "calcium and Pi lost by enamel can be more efficiently recovered if fluoride is still present in biofilm fluid" (2009 Cury). Thus, fluoride as an anti-caries agent is believed to both reduce demineralization and enhance remineralization of teeth.

Fluoride may be incorporated into oral care compositions through the use of various fluoride ion sources, such as sodium fluoride, amine fluoride, sodium monofluorophosphate (MFP), and stannous fluoride. Factors that can affect the anti-caries efficacy of fluoride include: fluoride ion source, concentration of the fluoride ion source, "frequency of use, duration of exposure, and method of delivery." (2006 Zero) For an oral care composition containing fluoride to be effective, it is necessary for the delivery of fluoride to provide 1) an effective or high fluoride concentration to be in contact with the tooth and plaque upon initial application and 2) oral fluids to retain fluoride following use. (2006 Zero)

Edgar states that "persistent elevation of salivary fluoride from baseline values around 1 µmol/L to perhaps 2-5 µmol/L ... is the true therapeutic factor in caries prevention." (1995 Edgar) According to Edgar, a MFP dentifrice, containing 1500 parts per million (ppm) of fluoride, can provide the aforementioned level of salivary fluoride. As stated previously, the final monograph entitled "Anticaries Drug Products for Over-the-Counter Human Use" specifies the biological and analytical tests that fluoride oral care compositions must pass to be considered safe and effective anti-caries products. The experimental anti-caries fluoride formulations must meet or exceed the relevant United States Pharmacopeia (USP) standard fluoride reference for performance and content in these biological and analytical tests. For dentifrices containing sodium fluoride in a gel or paste form, the dentifrice must contain 850 to 1150 ppm theoretical total fluorine and sodium fluoride of 0.188% to 0.254% weight/volume (w/v) with an available fluoride ion concentration of > 650 ppm. The experimental fluoride dentifrice must also meet or exceed the performance of the USP Standard Reference Fluoride Dentifrice in an Animal Caries Reduction Test and either one of the following tests, Enamel Solubility Reduction Test or Fluoride Enamel Uptake Test. For treatment rinses containing sodium fluoride in aqueous solution, the rinse may contain: 1) 0.02% (w/v) sodium fluoride at approximately pH 7 or 2) 0.05% (w/v) sodium fluoride at approximately pH 7.

### Stabilized Chlorine Dioxide

The term chlorine dioxide (ClO2) is widely used in the industry. Those skilled in the art will and do appreciate the various forms or variations thereof which are available to perform certain intended functions and purposes. U.S. Patent No. 3,271,242 describes a form of stabilized chlorine dioxide and a method of making the product, which is particularly useful in carrying out the present invention. The 1979 text Chlorine Dioxide, Chemistry and Environmental Impact of Oxychlorine Compounds, describes (aqueous) stabilized chlorine dioxide as follows:
the chlorine dioxide is practically completely transformed to chlorite. Dioxide is released upon acidification..." [Masschelein, 1979]

The term 'peroxy compounds' may substitute for 'percarbonates and perborates', referring to any buffer suitable for maintaining the pH and hence, the stability of the ClO2 in solution. The buffer is a necessary component, as the ClO2 is unstable at low pH. Once the solution reaches low pH or encounters an area of low pH, the stabilized ClO2 is released from solution and available for sanitation and oxidation. The composition of this invention requires chlorine dioxide stabilized using phosphate buffering, such as ..., but excluding MFP. The type and concentration of buffering compound may affect overall product stability as well as the stability of the pH of the composition.

Prior to its use in the 1950s, chlorine dioxide was known to have bactericidal properties (Masschelein, 1979). In U.S. Patent No. 2,451,897 Woodward, first established use of chlorine dioxide to eliminate the unpalatable taste in shrimp; thereafter, chlorine dioxide began to be used for its oxidative properties in various industries for different applications. Chlorine dioxide has been applied to bleaching cellulose fibers to facilitate the manufacture of wood pulp. Furthermore, chlorine dioxide has been used to disinfect water for public consumption with minimal effect on taste. Chlorine dioxide provides a beneficial alternative over other processes involving the use of ozone and bleach, due to the fact that chlorine dioxide costs less to use, creates less toxicity, and creates fewer chlorinated by-products (Masschelein, 1979).

In oral care products, the use of stabilized chlorine dioxide has been suggested as an active ingredient by a number of patents: U.S. Patents Nos. 4,689,215; 4,696,811; 4,786,492; 4,788,053; 4,792,442; 4,793,989; 4,808,389; 4,818,519; 4,837,009; 4,851,213; 4,855,135; 4,886,657; 4,889,714; 4,925,656; 4,975,285; 5,200,171; 5,348,734; 5,489,435; 5,618,550. US 4689215 claims the use of 0.005% to 0.2% stabilized chlorine dioxide solutions to reduce oral malodor through the oxidation of volatile sulfur compounds; it is also the first to suggest stabilized chlorine dioxide as an anticariogenic agent that acts by killing the cariogenic bacteria Streptococcus mutans. US 4696811 claims a method to reduce dental plaque through the topical malodor through the oxidation of volatile sulfur compounds; it is also the first to suggest stabilized chlorine dioxide as an anticariogenic agent that acts by killing the cariogenic bacteria Streptococcus mutans. US 4696811 claims a method to reduce dental plaque through the topical application of 0.005% to 0.2% of stabilized chlorine dioxide solution to the oral cavity which results in 99% kill and reduction of the cariogenic *S*. *mutans.* US 4786492 adds to '215 and '811 by providing a further method for stabilized chlorine dioxide reducing plaque through 90% bacterial kill of *S. mutans* and altering the ecological milieu of oral bacteria. Specifically '492 teaches that stabilized chlorine dioxide reduces plaque through its antibacterial properties that halt sucrose degradation and prevent dextran and levan formation, which deprives oral bacteria of nutrition. Furthermore, '492 also claims that, when used orally, a stabilized chlorine dioxide solution breaks double bonds of glucosyltransferases found in the oral cavity. Taken altogether, the properties of stabilized chlorine dioxide solution listed in '492 are recited to result in plaque reduction. US 4788053 teaches the incorporation and use of 0.005% to 0.2% stabilized chlorine dioxide in a paste to reduce plaque. US 4792442 recites the use of 0.005% to 0.2% stabilized chlorine dioxide solution to reduce plaque by oral irrigation of gingival crevices.

US 4793989 teaches the use of stabilized chlorine dioxide (SCD) to irrigate and soak a dental prosthetic device. It suggests that SCD reduces the degradation of proteins associated with dental prosthetics in part by reducing the motility and mitosis of all oral bacteria (including *Bacterioides melaninogenicus).*

US 4808389 is directed to a method to reduce gingivitis, periodontitis and permeability of submucosal tissue to bacterial invasion through the application of a 0.005% to 0.2% SCD solution in the oral cavity. It specifically mentions that SCD is capable of reducing these conditions through 90% kill and reduction of *S. mutans, Bacteroides gingivalis, Actinobacillus actinomycetumcomitans,* and gram positive bacteria.

US 5348734 further instructs the use of 0.02%-3.0% phosphate buffers, specifically disodium hydrogen phosphate, sodium dihydrogen phosphate, and trisodium phosphate, to increase the shelf life and efficacy of stabilized chlorine dioxide in dentifrice. The stabilized chlorine dioxide would prevent and treat dental diseases, like gingivitis, periodontitis, and dental caries, by reducing the number of microbial species in the mouth, such as *S. mutans, S. sanguis, Candida,* and oral pathogens. Streptococci, such as *S. mutan* and *S. sanguis,* facilitate the conversion of pellicle to plaque and the formation of biofilm.

The, prior art described above does not teach nor cite any affects on the dentin or tooth enamel or on the processes of demineralization or remineralization.

### Stabilized Chlorine Dioxide/Sodium Chlorite plus Fluoride Oral Care Products

In the prior art, there are many descriptions of oral care products containing fluoride ion sources and chlorine dioxide sources. US 6375933 teaches a composition and method for oral malodor involving a dual phase dentifrice. One phase contains the essential components of a zinc releasing compound and a chlorite releasing compound at neutral pH, with a fluoride ion source being optionally added in this first phase. The second phase is an acidic component, which contains an acidic substance such as a phosphoric acid. These two phases must be kept separate until the composition is dispensed and when applied to teeth.

US 5200171 instructs the use of 0.005-0.5% monofluorophosphate as the phosphate buffer to retard the escape of chlorine dioxide in 0.02-3.0% stabilized chlorine dioxide mouthwash and dentifrice at pH 6.0 to 7.4. The stabilized chlorine dioxide is enclosed as the agent that reduces *S. mutans* and it is not stated that the sodium monofluorophosphate contributes a demineralization or anticaries effect to the formulation or that the sodium monofluorophosphate is in a stable concentration to contribute a reduced demineralization or anticaries effect to the stabilized chlorine dioxide mouthwash or dentifrice. The purpose of the phosphate in the invention is to produce increased stability and shelf life of the stabilized chlorine dioxide compositions to reduce bacterial motility and kill to prevent and treat gingivitis, periodontitis and dental caries. The present invention specifically excludes the use of monofluorophosphates as buffers to stabilize pH due to their untoward affects on the overall stability of the composition.

US 6077502 (Witt, oral lozenge), US 6132702 (Witt, toothpaste), US 6235269 (Witt, dual phase toothpaste and non-abrasive ge1), US 6251372 (Witt, single phase oral rinse), US 6264924 (Witt, chewing gum) teach the use of oral care compositions containing the chlorite ion at basic pH (pH greater than 7) with levels of chlorine dioxide of less than 50 ppm. In these Witt patents, the chlorite ion, not chlorine dioxide, is the essential component to prevent and treat conditions of the oral cavity, including caries. In fact, these patents instruct that an ideal condition of these compositions is to contain a minimal amount of chlorine dioxide. All of these patents mention the use of the compositions and methods to treat and prevent oral diseases (including caries), but the specific scope of each of these patents is limited to periodontal disease, plaque, gingivitis, breath malodor, or teeth whitening. There is no mention in any of these patents that the compositions or methods enhance the efficacy of the remineralization of the tooth to treat and prevent caries. None of these patents present data that show that the suggested embodiments and formulations deliver effective amounts of chlorine dioxide, the chlorite ion and the fluoride ion when these two active ingredients are combined. All these compositions call for levels of chlorine dioxide of less than 50 ppm but there are no known studies that indicate that these formulations result in positive anti-caries effects equal to or significantly greater than the industry standard as well as antibacterial effects significantly greater than the industry standard compositions for fluoride or enhance the anti-caries effects of fluoride.

US 6132702 teaches a toothpaste for the treatment and prevention of breath malodor, gingivitis and conditions of the oral cavity (including caries) - the toothpaste is recited to contain at least 0.2% of the chlorite ion at a pH greater than 7 with less than 50 ppm of chlorine dioxide. US 6132702 further instructs the addition of 0.05% to about 0.3% of the fluoride ion to the claimed toothpaste.

US 6235269 details a dual phase toothpaste composition wherein the active chlorite ion is kept separate from a second oral carrier, until the invention is ready for use and then the two phases are mixed together to a final pH greater than 7.5 with less than 50 ppm of chlorine dioxide. The function for the dual phase composition in US 6235269 with 0.05% to 0.3% fluoride ion is to treat and prevent breath malodor. US 6251372 is directed to a single phase oral rinse containing at least 0.04% of the chlorite ion with a pH greater than 7.5 and less than 50 ppm of chlorine dioxide or free of chlorine dioxide. The purpose of this oral rinse composition is to treat and prevent breath malodor. US 6251372 further instructs the addition of 0.05% to 0.3% fluoride ion to the oral rinse composition, at a pH greater than 7.5 and free of or containing less than 5 ppm of chlorine dioxide. The patent states that the invention may be used to prevent and treat conditions of the oral cavity including caries. US 6264924 describes a composition for a chewing gum having a chlorite ion (1 mg to 6 mg) at a pH greater than 7 with less than 50 ppm chlorine dioxide present. The purpose for this composition is to treat and prevent periodontal disease, plaque, gingivitis, and breath malodor. US 6264924 also recites the inclusion of 0.05% to 0.3% of the fluoride ion to the chewing gum composition, at a pH greater than 7 and less than 50 ppm chlorine dioxide, however no indication is given for this composition. The patent states that the invention may be used to prevent and treat conditions of the oral cavity including caries.

US 6350438 expands on the previous Witt patents to include stable oral care compositions containing the chlorite ion for use by human and animal subjects for the treatment and prevention of oral cavity diseases, including caries. US 6350438 specifies that these oral care compositions are to contain 0.02% to 6.0% of the chlorite ion, with vitually no chlorine dioxide (less than 2 ppm) in the composition, and have a final pH greater than 7. According to this patent, the fluoride ion may be added to these compositions from 0.05% to 0.3%. US 6350438 further instructs the addition therapeutic agents (such as H2 antagonists, metalloproteinase inhibitors, cytokine receptor antagonists) to oral care compositions containing the chlorite ion. US 6350438 specifically refer to use of these compositions to treat periodontal disease via therapeutic actions imparted by the chlorite ion in combination with the various therapeutic components (including antimicrobial effects and the effect of aiding in the healing of periodontal tissue and regeneration). The text of US 6350438 indicates that these compositions are effective bactericides and bacteriostatic compounds, with the chlorite ion being selective for gram negative anaerobes associated with periodontal disease. (US 6350438 - Witt, general patent about treating oral cavity diseases using chlorite ion with vitually no chlorine dioxide - oral rinse and toothpaste are mentioned). This patent does specify the addition of the fluoride ion as an anticaries agent, but it teaches the use of the invention to prevent and treat a wide range of conditions of the oral cavity, including gingivitis, malodor, and caries. It does not teach the use of a chlorine dioxide source to enhance the anti-caries of fluoride.

US 6696047, recites oral care compositions containing 0.02% to 6.0% of the chlorite ion at alkaline pH which are essentially free of chlorine dioxide (less than 2 ppm of chlorine dioxide) - and the novelty of these compositions are that the prescribed formulations are stated to maintain stable amounts of the chlorite ion at 25°C for one year or 40°C for 3 months. According to the patent, stability is exhibited in the composition if the following is observed at 25°C for one year and/or 40°C for three months: the chlorite ion is delivered in efficacious amounts to the oral cavity, the composition does not degrade to form chlorine dioxide, and the composition does not degrade excipients (with a change in flavor being a major indicator of degradation). A quantifiable percentage of acceptable chlorite ion degradation from time zero is not explicitly defined in the patent for any embodiment. The sample formulations that are presented to show stability of various embodiments (presented in the table labeled 'Results of Stability Testing') are all prepared at alkaline pH 10. Chlorite ion concentration, pH and flavor concentration are measured to demonstrate stability of the formulations. The patent does not, however, present stability data that shows stability of the fluoride ion, when fluoride is included in combination with the chlorite ion in the cited dentifrice formulations (Example 3A through 3G Dentifrices) or in other embodiments. The compositions in US 66906047 are designed for human and animal subjects, and the text indicates that these compositions may be used to treat and prevent diseases of the oral cavity, including caries. However, no allegation is made that anti-caries effects of the composition exceed the industry standard or that the composition is able to enhence the anti-caries effects of fluoride.

US 7387774 teaches the use of two essential components, to enhance anti-caries protection and increase resistance to demineraliszation of the teeth, 1) soluble fluoride source to provide free fluoride ions and 2) phosphonate polymeric mineral surface active agent. According to the patent, in combination, these two essential components impart "enhanced protection of teeth against caries characterized by increased remineralization of teeth, increased fluoride deposition in teeth and increased resistance of teeth to acid demineralization while simultaneously providing anticalculus benefits." Enhance fluoride uptake is a significant mode of action that appears to result from the use of this composition. Other components are listed as additional agents that may be added to the composition to add certain benefits, sodium chlorite is specifically mentioned as a whitening agent to enhance teeth whitening. These additional agents are not required to impart the alleged anti-cavies benefits produced by the essential components.

A fluoride toothpaste sold under the trademark Oxyfresh contains stabilized chlorine dioxide and sodium fluoride (0.235%) containing zinc acetate. The inclusion of zinc in the formulation is cited as a key component in combination with stabilized chlorine dioxide to eliminate volatile sulfur compounds and reduce oral malodor. The formulation does not include the use of a phosphate buffer to help retard the escape of chlorine dioxide. There is no published scientific evidence that the Oxyfresh formulation delivers a stable and effective amount of the fluoride ion to ensure anticaries efficacy.

Summary of prior art patents directed to sodium chlorite oral care compositions (with or without mentions of flouride):
US 6077502 'Oral Care Compositions Comprising Chlorite and Methods'
US 6132702 'Oral Care Compositions Comprising Chlorite and Methods'
US 6235269 'Oral Care Compositions Comprising Chlorite and Methods'
US 6251372 'Oral Care Compositions Comprising Chlorite and Methods'
US 6264924 'Oral Care Compositions Comprising Chlorite and Methods'
US 6350438 'Oral Care Compositions Comprising Chlorite and Methods'
US 6696047 'Stable Oral Care Compositions Comprising Chlorite';
PCT/US2002/028324 'Stable Oral Care Compositions Comprising Chlorite'
US 7387774 ' Method of Enhancing Fluoridation and Mineralization of Teeth'
US 4689215 'Method and Composition for Prevention and Treatment of Oral Disease'
US 4696811 'Method and Composition for Prevention and Treatment of Oral Disease'
US 4786492 'Method and Composition for Prevention and Treatment of Oral Disease'
US 4788053 'Method and Composition for Prevention and Treatment of Oral Disease'
US 4792442 'Method and Composition for Prevention and Treatment of Oral Disease'
US 4793989 'Method and Composition for Prevention and Treatment of Oral Disease'
US 4808389 'Method and Composition for Prevention and Treatment of Oral Disease'
US 4818519 'Method and Composition for Prevention of Plaque Formation and Plaque Dependent Diseases'
US 4837009 'Method and Composition for Prevention of Plaque Formation and Plaque Dependent Diseases'
US 5200171 'Oral Health Preparation and Method'
US 5348734 'Oral Health Preparation and Method'
US 6375933 'Dual Component Dentifrice for Reducing Mouth Odors'

Other compositions are disclosed in US 6,582,682; US 2004/0126335; and WO 2004/077210

### SUMMARY OF THE INVENTION

An oral care composition in the form of a paste, gel, rinse, spray, powder, varnish or similar for reducing the biofilm attendant teeth to enhance the process of fluoride ions reducing demineralization and promote remineralization of teeth. The composition includes a chlorine dioxide source for releasing chlorine dioxide to create a bacteriocidal affect on the biofilm, a fluoride ion source wherein the fluoride ion is from sodium fluoride, enhanced by the chlorine dioxide to reduce demineralization and promote remineralization of the teeth and a buffer source to maintain the composition within its most effective pH range.

It is therefore a primary object of the present invention to provide a composition having anti-caries properties.

Another object of the present invention is to provide a composition for reducing demineralization and promoting remineralization of teeth.

Still another object of the present invention is to provide a composition for enhancing the effect of fluoride ions to reduce demineralization and promote remineralization of the teeth.

Yet another object of the present invention is to provide a single phase composition for reducing demineralization and promoting remineralization of the teeth.

A further object of the present invention is to provide a composition that exceeds established standards for reducing demineralization and promote remineralization of the teeth.

A still further object of the present invention is to provide a composition having a significant shelf life for reducing demineralization and promote remineralization of the teeth.

A yet further object of the present invention is to provide a method for enhancing the reduction of demineralization and promotion of remineralization of the teeth.

These and other object of the invention will become apparent to those skilled in this art as the description thereof proceeds.

### DETAILED DESCRIPTION OF THE PREFERRED EMBODIMENTS

The present invention teaches oral care compositions, including paste, gel, rinse, spray, powder, varnish or similar, in the form of a 1) chlorine dioxide source selected from a group consisting of stabilized chlorine dioxide, 2) fluoride ion source from sodium fluoride, and a 3) buffering system to achieve a specific pH of the final composition, the buffers may include acetate, citrate, phosphate buffers, and other buffers known to those skilled in the art. The oral care compositions of the present invention include the following fundamental components:
a) Concentration of 0.005% to 0.800% of stabilized chlorine dioxide by weight of the final composition;
b) Concentration of the fluoride ion source , sodium fluoride, that provides a fluoride ion concentration of 45 ppm to 5000 ppm by weight of the final composition;
c) A buffering system (described in the specification below) that achieves a pH of the final composition in a range in a concentration of 6.0 to 7.4.

The present invention teaches methods to deliver the composition to the human oral cavity (including but not limited to the teeth, tongue, gingiva, and saliva) through topical application of the composition to the human oral cavity, at least once daily.

The composition and method of the present invention is believed to enhance the anti-caries effects of fluoride as it is believed that the stabilized chlorine dioxide does not interfere with the normal uptake of fluoride by the enamel and also it is believed that the stabilized chlorine dioxide is able to disrupt dental biofilm of the teeth to preclude cariogenic bacteria from creating the acidic environment necessary for caries progression and caries development.

The composition is not intended to be a dual phase composition and does not rely on the end user mixing ingredients immediately prior to use to produce chlorine dioxide, as described in US Patents Nos: 4084747, 4330531, and 5738840.

The composition of the present invention includes fundamental components, optional components, and has various methods of delivery and treatment regimens, and a specific mechanism of action described in more detail below.

One fundamental component of the oral care compositions of the present invention is a chlorine dioxide source. The term chlorine dioxide source is used to describe a water soluble chlorine dioxide source that 1) contains chlorine dioxide and/or 2) stabilized chlorine dioxide in an aqueous solution and able to form chlorine dioxide when the chlorine dioxide source is delivered to the human oral cavity (including but not limited to the teeth, gingiva, tongue and saliva). The chlorine dioxide source is selected from the group consisting of stabilized chlorine dioxide. For the present invention, the instructed levels of the chlorine dioxide source in the oral care compositions are 0.005% to 0.800% weight/weight (w/w) or weight/volume (w/v) stabilized chlorine dioxide.

The purpose of the chlorine dioxide source is deliver chlorine dioxide to the human oral cavity to disrupt the dental biofilm that contributes to caries development and progression, and thereby preclude cariogenic bacteria from creating the acid environment necessary for caries development and progression. Therefore, the chlorine dioxide source enhances the anti-caries effects of fluoride. The selection of this range for the concentration of stabilized chlorine dioxide in the present invention is based on prior work investigating the properties of stabilized chlorine dioxide, at various concentrations, against known cariogenic bacteria (such as *Streptococcus mutants, Lactobacillus,* and *Streptococcus sanguis*) and against biofilms. US 4689215 describes the ability of a 0.020% (w/v) or 200 ppm stabilized chlorine dioxide oral rinse solution (at pH 6) to achieve 98.1 to 99.5% in vitro kill of the cariogenic bacteria *S. mutans* after treatment for 10 and 20 seconds, respectively. In 2001, Grootveld et. al. published a study that reported the effect of a 0.100% (w/v) stabilized chlorine dioxide oral rinse to reduce levels of *Streptococcus mutans, lactobacilli,* and *Candida albicans* in human saliva. The experimental group of 33 subjects rinsed with 20 ml of a 0.100% (w/v) stabilized chlorine dioxide oral rinse three times daily for 60 seconds, and continued this regimen for 14 days. An independent negative control group of 10 subjects rinsed with 20 ml of mineral water for 60 seconds, and continued this regimen for 14 days. Saliva was collected from both groups at baseline and 14 day follow up. The stabilized chlorine dioxide oral rinse was shown to significantly reduce bacterial counts of *S. mutans* and lactobacilli in saliva, however the reduction of *C*. *albicans* was not found to be significant. (Grootveld et. al., 2001) It is predicted that similar bactericidal effects would be observed in compositions of the present invention containing 0.020% (w/v) stabilized chlorine dioxide, with the level and rate of bacterial kill decreasing as the concentration of stabilized chlorine dioxide decreases.

PCT/US2008/055154 describes the bacteriostatic and bactericidal properties of the use of oral care compositions containing stabilized chlorine dioxide in the range of 0.005%-0.800% (w/v). This publication describes the bactericidal properties observed in in vitro kill of known anaerobic, aerobic, facultative gram-negative and gram positive oral bacteria present in mixed microbial communities. The bacteria tested and presented include: *Porphyromonas gingivalis, Actinomyces odontolyticus* and *A. viscosus, Prevotella intermedia, Fusobacterum nucleatum, Micromonas micros, Streptococcus sanguis* and *S. oralis.* A key observation in this work was the susceptibilities of the bacterium to chlorine dioxide do not exhibit a linear antibacterial effect to increasing chlorine dioxide concentration in stabilized chlorine dioxide rinses rangiing from 0.1 % SCD to 0.4% SCD. At 0.4% SCD and above, a significant increase in bacterial kill in a polymicrobial suspension was observed. In addition to this observation, there was also prior work which showed that stabilized chlorine dioxide solutions were able to decontaminate biofilms that occurred in dental unit waterlines (DUWL). Wirthlin showed that an undisclosed concentration of buffer-stabilized chlorine dioxide solution was able to decontaminate DUWL biofilms with results that were similar and in some cases better than a freshly mixed chlorine dioxide treatment. (Wirthlin 2003). Further to this work is the observation of the effect of stabilized chlorine dioxide on known oral bacteria found in biofilm. Villhauer et. al., (2009) presented evidence of bactericidal activity of stabilized chlorine dioxide against polymicrobial biofilms. The bacteria included in the experiment were specific to periodontal pathogens with some pathogens known to be cariogenic such as *Actinomyces viscosus, Streptococcus sanguinis, Fusobacterium nucleatum, Peptostreptococcus micros,* and *Porphyromonas gingivalis.* Cultured biofilms were exposed to one minute regimens of a 0.500% (w/v) stabilized chlorine dioxide oral rinse. The results of this study showed that multiple exposures to the oral rinse: 1) reduced bacterial counts of *S. sanguis* by 2-3 logs, 2) eliminated almost all counts of *P. gingivalis, P. micros,* and *F. nucleatum,* and 3) had little to no effect on counts of *A. viscosus.* Single exposure to the oral rinse yielded no significant bacterial counts. Based on these prior observations, an upper limit of 0.800% (w/w or w/v) of stabilized chlorine dioxide seems reasonable to achieve the bactericidal and disruptive biofilm properties of stabilized chlorine dioxide. It is unknown whether oral care compositions containing stabilized chlorine dioxide, outside the ranges specified by the present invention, will have a significant increased effect on bacterial kill or disrupting biofilms, therefore the specified limits for the concentration of the chlorine dioxide source are instructed for the present invention based on the current understanding of these properties of stabilized chlorine dioxide.

Stabilized chlorine dioxide (SCD) is taught as a chlorine dioxide source in the present invention based on prior art that demonstrates that a stabilized chlorine diozide source is able to generate chlorine dioxide when SCD is present in the final composition in a concentration taught by the present invention. A 0.100% (w/v) stabilized chlorine dioxide oral rinse was shown to contain very low concentrations of chlorine dioxide at pH of 6.5 (1997 Lynch). It is believed that the mechanism for generation of the chlorine dioxide from stabilized chlorine dioxide is due to the degradation of specific amino acids by stabilized chlorine dioxide, which results in the generation of chlorine dioxide. Therefore, it is expected that a similar generation of chlorine dioxide would be formed by the levels of the chlorine dioxide source and pH range instructed by the present invention.

A second fundamental component of the oral care compositions of the present invention is a fluoride ion source. The term fluoride ion source is used to describe a water soluble fluoride ion source that is able to provide available, free fluoride ions to the human oral cavity (including but not limited to the teeth, gingiva, tongue, and saliva). The fluoride ion source of the present invention is sodium fluoride (NaF).

The purpose of the fluoride ion source in the present invention is to make fluoride ions available to the human oral cavity which will impart the known anti-caries effects of fluoride, specifically to reduce demineralization for the teeth and to enhance remineralization of the teeth. (2008 Garcia-Godoy, 2009 Cury) The fluoride ion source of the present invention is sodium fluoride (NaF). Stannous fluoride (SnF₂) is excluded as a fluoride ion source for the present invention. The exclusion of stannous fluoride is due to the known oxidative properties of chlorine dioxide and stannous fluoride that presumably degrade the stability of chlorine dioxide. Chlorine dioxide is a powerful oxidizer, and stannous (Sn²⁺) fluoride is known to be oxidized in aqueous solutions by oxygen to the stannic state (Sn⁴⁺), with the rate of oxidation increasing as the pH of the solution is more acidic. (1997 Lynch, 1994 Denes). It is believed that when chlorine dioxide is present in or liberated from stabilized chlorine dioxide in the present invention, which is slightly acidic, the chlorine dioxide oxidizes the stannous (Sn²⁺) to the stannic oxidative state (Sn⁴⁺). It is further thought that this oxidation reaction renders the fundamental component of the present invention, chlorine dioxide, unstable. This belief is substantiated by the stability results presented in Table 1 below, which illustrates the stability of stabilized chlorine dioxide when combined with various fluoride ion sources including stannous fluoride.

**Table 1. Three (3) Month Intermediate and Accelerated Stability Testing of Dentifrice Formulations**

| *Stability of Stabilized Chlorine Dioxide in Dentifrice Formulations Containing Various Fluoride Ion Sources* | | | | | | | |
|---|---|---|---|---|---|---|---|
| | | | | | | | |

| | | *pH* | *% ClO₂* | *Fluoride (ppm or %)* | *Fluoride Ion Source* | | |
|---|---|---|---|---|---|---|---|
| *Initial* | *Sample A* | 7.40 | 0.12 | 1287 | Sodium Fluoride | | |
| | *Sample B* | 7.26 | 0.12 | 1.10% | Sodium Monofluorophosphate | | |
| | *Sample C* | 7.20 | 0.12 | 1090 | Stannous Fluoride | | |
| | | | | | | | |

| | | *Intermediate pH* | *Intermediate % ClO₂* | *Intermediate Fluoride (ppm or %)* | *Accelerated pH* | *accelerate % ClO₂* | *Accelerated Fluoride (ppm or %)* |
|---|---|---|---|---|---|---|---|
| *Month 1* | *Sample A* | -- | 0.1 | 1326 | -- | 0.04 | 1316 |
| | *Sample B* | -- | 0.1 | -- | -- | 0.03 | 0.73% |
| | *Sample C* | -- | 0.03 | 1136 | -- | 0.03 | 1109 |
| | | | | | | | |
| *Month 2* | *Sample A* | 7.46 | 0.06 | 1306 | 7.37 | 0.01 | 1309 |
| | *Sample B* | 7.2 | 0.06 | -- | 7.06 | 0 | 0.78% |
| | *Sample C* | -- | -- | -- | -- | -- | -- |
| | | | | | | | |
| *Month 3* | *Sample A* | 7.49 | 0.04 | 1301 | 7.44 | 0 | 1280 |
| | *Sample B* | 7.17 | 0.03 | -- | 7.06 | 0 | - |
| | *Sample C* | -- | -- | -- | -- | -- | -- |

| | | | | | | | |
|---|---|---|---|---|---|---|---|
| Table 1. Table 1 provides stability data for three different fluoride and stabilized chlorine dioxide dentifrice formulations. Intermediate conditions are storage conditions of 30°Celsius. Accelerated conditions are 40°Celsius and 70% Relative Humidity. Such conditions are used to forecast the degradation of the active ingredient(s) in a drug product that occurs in real time. There are guidance documents from various regulatory and governing bodies that further discuss the purpose of stability testing and how to evaluate results of stability testing of new and existing drugs. (2003 International Conference on Harmonisation of Technical Requirements for Registration of Pharmaceuticals for Human Use) '--' indicates that this attribute was not tested at this time point. | | | | | | | |

Table 1 displays data from three month stability testing of the three dentifrice formulations placed under intermediate and accelerated storage conditions. The purpose of the stability test was to evaluate the stability of different dentifrice formulations and determine the effect that different fluoride ion sources have on the stability of stabilized chlorine dioxide. At time zero, all of the formulations had an initial stabilized chlorine dioxide concentration of 0.12% (w/w). Under intermediate conditions, the formulation containing stannous fluoride (Sample C) presented the greatest chlorine dioxide degradation at 'Month 1' having lost about 75% of the initial concentration of stabilized chlorine dioxide, compared with the formulations containing sodium fluoride and sodium monofluorophosphate which only lost about 16.7% of the initial concentration of stabilized chlorine dioxide. The study was discontinued for the stannous fluoride formulation (Sample C) after Month 1, but both the sodium fluoride and sodium monofluorophosphate formulations (Sample A and Sample B) were kept on stability testing through three months and showed nearly the same stability results for stabilized chlorine dioxide concentration under both intermediate and accelerated conditions. Given the results above in Table 1, it substantiates the belief that stannous fluoride should not be included as a fluoride ion source in the present invention because it appears to degrade stabilized chlorine dioxide more rapidly than sodium fluoride or sodium monofluorophosphate.

The concentration of the fluoride ion source instructed by the present invention should provide an available fluoride ion concentration of 45 to 5000 ppm of the fluoride ion in the final composition. The basis for the instructed range of the fluoride ion source in the present invention is primarily based on accepted levels of the fluoride ion known to those skilled in the art to provide safety and anti-caries efficacy of different embodiments of the final composition. As previously stated, the United States (US) FDA anti-caries monograph for over-the-counter drug products, USC Title 21 Parts 310, 355, and 369, states specifications for the fluoride ion content of oral care compositions that are considered to be safe and effective against caries. Based on the specifications of this FDA monograph, it seems reasonable to have a lower limit of 45 ppm of the fluoride ion in the present invention to provide an amount of the fluoride ion necessary for anti-caries efficacy. For an oral rinse containing sodium fluoride to be acknowledged as a safe and effective anti-caries product, the FDA monograph specifies a minimum concentration of sodium fluoride of 0.02% (at pH 7) which would provide approximately 90 ppm of the fluoride ion. (1995 Food and Drug Administration) Likewise, for an oral rinse containing sodium fluoride to be considered as a safe and effective anti-caries product, the FDA monograph specifies a maximum of 0.05% sodium fluoride (at pH 7) which would provide approximately 226 ppm of the fluoride ion. In Europe, where fluoride products are regulated as cosmetic products, there is a maximum limit of 1500 ppm of sodium fluoride, which would provide approximately 675 ppm of the fluoride ion, in all types of oral hygiene products, including oral rinse. Therefore, for an oral rinse embodiment of the present invention, the preferred concentration of the fluoride ion is 45 to 800 ppm of the fluoride ion, with the most preferred concentration of the fluoride ion being 90 ppm to 675 ppm. (1995 FDA, 1999 European Commission)

Values greater than 1500 ppm and up to 5000 ppm of the fluoride ion, that are specified by the present invention, are instructed strictly for use of the oral composition by professional dentists and not by the public as over the counter drugs. At levels above 5000 ppm, oral care compositions lean toward toxicity and individuals receiving oral care compositions with levels above 1500 ppm of the fluoride ion should be both administered the composition and monitored by a dental or medical professional. Thus, the levels of the fluoride ion specified by the present invention seem reasonable to provide safe and effective anti-caries compositions, based on known standards for oral care compositions containing fluoride.

A third fundamental component of the oral care compositions of the present invention is a buffering system. The buffering system is selected from a group including acetate, citrate, phosphate buffers (including trisodium phosphate, sodium phosphate monobasic, and sodium phosphate dibasic), and other buffers known to those skilled in the art. Lactic acid, pyruvic acid, and other acids formed by cariogenic bacteria, known to those in the art as major acidic components in caries development and progression, are however excluded for use in the present invention. The inclusion of the buffering system in the present invention is to create a pH of the final composition of about pH 6.0 to 7.4. The purpose of the buffering system is to prevent or retard the escape of chlorine dioxide, the conversion of stabilized chlorine dioxide into chlorine dioxide gas, prior to use of the present invention by the consumer. The buffering system is also present to achieve a specific pH range for the final composition. The pH range of the present invention is taught to be 6.0 to 7.4, due to observations made from prior art and the known properties of stabilized chlorine dioxide. Prior art teaches that a pH of approximately 7.5 or higher (and in some cases pH 7 or higher) creates more stable oral care compositions for the chlorite ion, a known component of stabilized chlorine dioxide (US 6077502, US 6132702, US 6235269, US 6251372, US 6350438, US 6696047). Oral care compositions containing the chlorite ion are described to be so stable at or above pH 7.5 that these compositions are claimed to be essentially free of chlorine dioxide (less than 50 ppm). Prior work investigating the bactericidal properties of stabilized chlorine dioxide bears out this assumption, as it was shown that the bactericidal activity of stabilized chlorine dioxide decreased as the pH was increased from pH 3.5 to 8.6. At pH 8.6, when the bactericidal effect decreased, the dominant chemical species was chlorite rather than chlorine dioxide, and from this observation "free chlorine dioxide" was assumed to be "the active disinfecting species." (1988 Harakeh) Thus at more alkaline pH, above pH 7.4, it is thought that the composition would not be a chlorine dioxide source since the composition is more stable for the chlorite ion and subsequently the composition would release less chlorine dioxide, which would therefore degrade the enhanced anti-caries effect taught by the present invention.

Conversely, when the pH of a stabilized chlorine dioxide composition becomes acidic, it is known to those skilled in the art that stabilized chlorine dioxide becomes unstable and results in increased conversion of stabilized chlorine dioxide to chlorine dioxide (in the gaseous form). Thus, at more acidic pH, it is thought that the duration of effectiveness of an oral care composition containing stabilized chlorine dioxide decreases as chlorine dioxide is lost as a gas, prior to application of the composition. It is therefore believed that a reasonable lower limit for pH of the present invention is about pH 6.0 and prior art substantiates this belief because US 4689215 teaches that a composition containing stabilized chlorine dioxide retains its bactericidal properties at pH 6.0. It is unknown whether oral care compositions containing stabilized chlorine dioxide can maintain its bactericidal properties or ability to disrupt biofilm at pH values below 6.0.

Further to the discussion regarding buffering systems, prior art substantiates the reasoning stated above regarding the pH range of pH 6.0-7.4 that is designated for the present invention. US 5348734 provides stability data for stabilized chlorine dioxide in various phosphate buffer solutions including trisodium phosphate, sodium phosphate monobasic, and sodium phosphate dibasic. '734 shows that at pH 6.8-7.5, stabilized chlorine dioxide is stable in these various buffers over 28 days.

### Excipients

It has been stated that oral carriers or oral excipients (optional components) may be added to the fundamental components of the present invention. The intent of the addition of these oral carriers or oral excipients is: to provide cosmetic attributes (such as flavor), to impart physical attributes (such as a thickened feel), to enable the stable combination or binding of the fundamental components, or mixtures thereof. The addition of these oral carriers or oral excipients provide cosmetic or physical attributes that are not possible with the fundamental components alone. These oral carriers may include, but are not limited to, the following classes of materials: sweetening agent(s) (sucralose, sodium saccharin, or similar), abrasive agent(s) (Sident 9, Sident 10, or similar), thickening or gelling agent(s) (Carrageenan gels, Carbomer 940, CMC 7H3SF, hydrated silicas, or similar), coloring agent(s) (Titanium Dioxide or similar), flavoring agent(s) (peppermint oil, spearmint oil or similar) or humectant(s) (Glycerol, Mannitol, Sorbitol, or similar).

### Mechanism of Action

The present invention contains a fluoride ion source and a chlorine dioxide source with respective chemical and physical properties previously discussed. It is believed that the levels of stabilized chlorine dioxide taught by the present invention do not interfere with the uptake of fluoride by the tooth enamel. This belief is demonstrated by the results of an Enamel Fluoride Uptake Test shown in Table 2 below:

**Table 2. Enamel Fluoride Uptake Test Results**

| Enamel Fluoride Uptake Test | | | | | |
|---|---|---|---|---|---|
| Sample ID | n | Statistical Values | Pre-Treatment Fluoride Level (ppm) | Post-Treatment Fluoride Level (ppm) | Fluoride Uptake (ppm) |
| L | 18 | Mean | 47.77 | 941.10 | 893.33 |
| | | SD | 9.82 | 107.13 | 104.23 |
| | | SEM | 2.32 | 25.25 | 24.57 |
| | | | | | |
| M | 16 | Mean | 49.72 | 912.73 | 863.01 |
| | | SD | 17.03 | 57.01 | 51.40 |
| | | SEM | 4.26 | 14.25 | 12.85 |
| | | | | | |
| N | 16 | Mean | 46.71 | 184.49 | 137.78 |
| | | SD | 11.29 | 29.06 | 21.17 |
| | | SEM | 2.82 | 7.27 | 5.29 |
| | | | | | |
| *Sample L* (Positive Control): USP Reference Fluoride Dentifrice with 0.243% Sodium Fluoride (w/w) and Silica | | | | | |
| *Sample M* (Experimental): Dentifrice with 0.24% (w/w) Sodium Fluoride and 0.125% (w/w) Stabilized Chlorine Dioxide with 1.54% phosphate buffer | | | | | |
| *Sample N* (Negative Control): Placebo non-Fluoride Dentifrice with 0.125% (w/w) Stabilized Chlorine Dioxide Dentifrice with 1.44% phosphate buffer | | | | | |
| Data from one specimen in Samples M and N, respectively, were rejected as outlier data. | | | | | |
| Data from one specimen in Samples M and N, respectively, were rejected due to technician error. | | | | | |

| | | | | | |
|---|---|---|---|---|---|
| Table 2. The Enamel Fluoride Uptake Test performed on Sample L, Sample M, and Sample N was a modified version of FDA Method #40, with one modification being the method for the formation of the caries-like (subsurface) lesion. Human teeth were used as specimens for this Enamel Fluoride Uptake test. The *'Pre-Treatment Fluoride'* value is the indigenous amount of fluoride in the specimen. The *'Post-Treatment'* value is the amount of fluoride measured in the specimen after the specimen was exposed to the specific dentifrice. The *'Fluoride Uptake'* is the resulting value when *'Pre-Treatment Fluoride'* is subtracted from *'Post-Treatment Fluoride.'* | | | | | |

The purpose of the Enamel Fluoride Uptake Test is to evaluate the effect that a dentifrice has on the uptake of fluoride into incipient enamel caries-like lesions. The results of the Enamel Fluoride Uptake Test show that there was no significant difference (p > 0.05%) between the *'Fluoride Uptake'* calculated for the USP reference standard for a sodium fluoride dentifrice (Sample L) and the experimental sodium fluoride and stabilized chlorine dioxide toothpaste (Sample M which is an embodiment of the present invention). Therefore it is believed that the results presented in Table 2 substantiate the belief that the levels of stabilized chlorine dioxide taught in the present invention do not interfere with the normal uptake of fluoride by the tooth enamel, and it is further expected that the inclusion of the stabilized chlorine dioxide in different embodiments of the present invention will not interfere with the normal uptake of fluoride by the tooth enamel.

Based on previously cited work documenting the effect of stabilized chlorine dioxide on kill of known cariogenic bacteria in biofilm and kill of known cariogenic bacteria *in vitro,* it is believed that the instructed levels of stabilized chlorine dioxide in the present invention disrupt the dental biofilm of human teeth which results in a preclusion of cariogenic bacteria (such as *S. mutans, S. sanguis,* and *Actinomyces viscosus*) in the dental biofilm. (US 4689215, 2001 Grootveld, PCT/US/2008/055154, 2001 Wirthlin, 2009 Villhauer). It is further thought that this disruption of dental biofilm thereby makes these cariogenic bacteria less capable of creating the acidic environment necessary for caries development and caries progression. As previously stated, it is also believed that the stabilized chlorine dioxide allows for normal uptake of fluoride by the enamel of the tooth (demonstrated through data shown in Table 2). As a result of these actions thought to occur due to the presence of stabilized chlorine dioxide in oral care compositions of the present invention, it is believed that the present invention enhances the anti-caries effects of fluoride. The belief in the enhancement of the anti-caries effect of fluoride is substantiated by the results observed in a Rat Caries Test performed on an embodiment of the present invention. The results of the Rat Caries Test are discussed in more detail below (Table 3a and Table 3b).

**Table 3a. Rat Caries Test Rat Caries Test**

| | | *Rat Caries Test Results for Sodium Fluoride Plus Stabilized Chlorine Dioxide Dentifrice* | | | | *Rat Caries Test Results from Warrick, et al 1999* | | | | | |
|---|---|---|---|---|---|---|---|---|---|---|---|
| | | *Placebo* | *USP Ref* | *Test* | | *Placebo* | *Test* | *Test* | *Test* | *Test* | *Test* |
| | ***Active Ingredient in Formulation*** | *0.125% ClO₂ (w*/*w)* | *NaF* | *NaF*/ *0.125% ClO₂ (w*/*w)* | | *KCl (grp C)* | *NaF (grp F)* | *Amine F (grp D)* | *NaF (grp A)* | *NaF (grp E)* | *NaF (grp B)* |
| | ***Intended F Content (ppm)*** | - | 1100 | 1100 | | - | 1400 | 1400 | 1400 | 1400 | 1400 |
| | | | | | | | | | | | |
| *Smoot h Caries* | *Enamel Involvement* | 22.15 | 15.20 | 9.40 | | 15.50 | 12.03 | 10.92 | 10.82 | 9.97 | 8.81 |
| | *Slight Dentinal Involvement* | 1,70 | 3.63 | 1.35 | | 3.19 | 2.86 | 1.92 | 2.44 | 2.28 | 1.25 |
| | *Moderate Dental Involvement* | 0.05 | 0.68 | 0.03 | | 0.50 | 0.39 | 0.36 | 0.22 | 0.28 | 0.00 |
| | *% Reduction of Enamel Involvement* | -- | 31.4% | 57.6% | | -- | 22.4% | 29.5% | 30.2% | 35.7% | 43.2% |
| | | | | | | | | | | | |
| *Sulcal Caries* | *Enamel Involvement* | 25.43 | 26.48 | 21.10 | | 16.94 | 11.97 | 10.03 | 9.97 | 8.67 | 10.50 |
| | *Slight Dentinal Involvement* | 0.95 | 2.93 | 0.95 | | 3.38 | 4.78 | 3.22 | 3.67 | 2.42 | 2.64 |
| | *Moderate Dentinal Involvement* | 0.18 | 0.85 | 0.15 | | 0.14 | 0.11 | 0.03 | 0.08 | 0.17 | 0.00 |
| | *% Reduction of Enamel Involvement* | -- | -4.1% | 17.0% | | -- | 29.3% | 40.8% | 41.1% | 48.8% | 38.0% |
| | | | | | | | | | | | |
| *Total Caries* | *Enamel Involvement* | 47.58 | 41.68 | 30.50 | | 32.44 | 24.00 | 20.94 | 20.25 | 18.64 | 19.31 |
| | *Slight Dentinal Involvement* | 2.65 | 6.56 | 2.30 | | 7.03 | 7.64 | 5.14 | 6.11 | 4.69 | 3.89 |
| | *Moderate Dentinal Involvement* | 0.23 | 1.53 | 0.18 | | 0.50 | 0.39 | 0.36 | 0.22 | 0.28 | 0.00 |
| | % Reduction of Enamel Involvement | -- | 12.4% | 35.9% | | -- | 26.0% | 35.5 | 37.6% | 42.5% | 40.5% |

| | | | | | | | | | | | |
|---|---|---|---|---|---|---|---|---|---|---|---|
| Table 3a. The Indiana Rat Caries Model described by Warrick, *et al,* using Spraque-Dawley rats, was performed in both of the study results displayed in Table 3a and Table 3b. (1999 Warrick) The Indiana Rat Caries Model is similar to FDA Method #37, with one major modification being that the caries lesions of the mandibular and maxillary molars are scored using the Keyes' Method instead of the Francis' Hypominerlized Area (HMA) model. The severity of a caries lesion in the Keyes method is measured according to lesion type (buccal, lingual, succal and proximal) and by assessing the severity of caries by distinguishing among enamel involvement, slight dentinal involvement, moderate dentinal involvement and extensive dental involvement. The Keyes' Method is familiar to those skilled in the art. (1958 Keyes) The *'% Reduction of Enamel Involvement'* was calculated by comparing the *'Enamel Involvement'* scores of the Test or USP pastes to *the'Enamel Involvement'* score for the Placebo, in each respective category. For example, for *'Total Caries'* the placebo score for *'Enamel Involvement' was* 47.58 and the *'Enamel Involvement'* test score for the NaF/ClO₂ dentifrice was 30.50, therefore there was a 35.9% reduction of enamel involvement for the NaF/ClO₂ toothpaste compared with the placebo formulation. '--' indicates that this attribute was not tested at this time point. | | | | | | | | | | | |

The purpose of the Rat Caries Test was to determine the effect a dentifrice has on the formation of caries in the rat. There was an unanticipated and unexpected result in the rat caries test scores displayed in Table 3a, which is the observation that the Sodium Fluoride and Stabilized Chlorine Dioxide (NaF/ClO₂) dentifrice (an embodiment of the present invention) outperformed the USP reference standard for NaF dentifrice in *'Total Caries -* '*% Reduction of Enamel Involvement. '* The NaF/ClO₂ had a *'Total Caries - % Reduction of Enamel Involvement'* score of 35.9% compared with a *%* reduction score of 12.4% for the USP reference standard, which is nearly a three times greater *%* reduction in enamel involvement by the NaF/ClO₂ dentifrice. Furthermore, the *'Total Caries - % Reduction of Enamel Involvement'* score for the NaF/ClO₂ dentifrice of 35.9% was comparable with the *'Total Caries - % Reduction of Enamel Involvement'* observed by the dentifrices tested in Warrick *et al* (26.0%, 35.5%, 37.6%, 42.5%, 40.5%, respectively) and it should be noted that all the dentifrices tested in the Warrick, study had higher levels of fluoride (1400 ppm) than the NaF/ClO₂ dentifrice (1100 ppm). Therefore, based on this evidence it is believed that the present invention enhances the anti-caries effect of fluoride.

The data in Table 3a for the '% *Reduction of Enamel Involvement'* only looks at the *'Enamel'* scores and does not evaluate caries involvement beyond the enamel other than presenting the values for *'Slight Dentinal Involvement'* and *'Moderate Dentinal Involvement'* (for Warrick, *et al* and the NaF/ClO₂ tests). To further demonstrate the effect of the dentifrices (from Table 3a) on caries progression, Table 3b below takes into consideration the amount of dentinal involvement relative to enamel involvement in order to assess the effect of each dentifrice on caries progression.

**Table 3b. Rat Caries Study - Caries Progression (Percentage of Enamel Source)**

| | | | | | | Warrick et al 1999 - Caries Progression | | | | | |
|---|---|---|---|---|---|---|---|---|---|---|---|
| | *Active Ingredient in Formulation* | ClO₂ | NaF | NaF/ClO₂ | | KCl (grp C) | NaF (grp F) | Amine F (grp D) | NaF (grp A) | NaF (grp E) | NaF (grp B) |
| | | | | | | | | | | | |
| Smooth | Slight Dentinal Involvement | 7.7% | 23.9% | 14.4% | | 20.6% | 23.8% | 17.6% | 22.6% | 22.9% | 14.2% |
| | Moderate Dentinal Involvement | 0.2% | 4.5% | 0.3% | | 3.2% | 3.2% | 3.3% | 2.0% | 2.8% | 0.0% |
| | | | | | | | | | | | |
| Sulcal | Slight Dentinal Involvement | 3.7% | 11.1% | 4.5% | | 20.0% | 39.9% | 32.1% | 36.8% | 27.9% | 25.1% |
| | Moderate Dentinal Involvement | 0.7% | 3.2% | 0.7% | | 0.8% | 0.9% | 0.3% | 0.8% | 2.0% | 0.0% |
| | | | | | | | | | | | |
| Total | Slight Dentinal Involvement | 5.6% | 15.7% | 7.5% | | 21.7% | 31.8% | 24.5% | 30.2% | 25.2% | 20.1% |
| | Moderate Dentinal Involvement | 0.5% | 3.7% | 0.6% | | 1.5% | 1.6% | 1.7% | 1.1% | 1.5% | 0.0% |

| | | | | | | | | | | | |
|---|---|---|---|---|---|---|---|---|---|---|---|
| Table 3b. The percentages in each box represent the percentages of caries progression and is calculated by dividing the respective *'Slight Dentinal lnvolvement' or 'Moderate Dentin Involvement'* scores by the *'Enamel Involvement'* scores all presented in Table 3a. | | | | | | | | | | | |

One unanticipated result presented in Table 3B is that the dentifrices with stabilized chlorine dioxide (ClO₂) both the placebo ClO₂ dentifrice and NaF/ClO₂ dentifrice, seem to do a much better job of preventing decayed caries progression into the inner tissue of the tooth. The ClO₂ pastes have a 5.6 and 7.5% progression categorized as '*Slight Dentinal Involvement,'* while the dentifrice without ClO₂ have 21.7, 31.8, 24.5, 39.2, 25.2, 20.1 and 15.7%. Further, the ClO₂ dentifrices have 0.5 and 0.6% progression categorized as *'Moderate Dentinal Involvement, '* while the dentifrices without ClO₂ have 1.5, 1.6, 1.7, 1.1, 1.5 and 3.7%.

For various reasons, the FDA has decreed the rat caries test as one of the standard biological tests that are sufficient to establish that a new fluoride dentifrice cariostatic in humans. The standard for performance is measured against a known USP fluoride reference standard dentifrice. (USC Title 21 Parts 310, 355, 369) Stookey also evaluated four coronal caries models to determine whether these pre clinical, animal models were able to forecast the clinical anti-caries effectiveness of fluoride dentifrices in humans, including that Indiana rat caries data presented above in Table 3a and Table 3b. A number of observations were made about this Indiana rat caries model: 1) it relies on infection of rats with known cariogenic bacteria that infect humans to form caries, 2) the caries condition induced in the model responds to the addition of fluoride, 3) and typically the "fluoride dose response" is similar to that which "has been observed in human clinical trials with fluoride dentifrice." (1995 Stookey) Taken altogether, it is believed that these rat caries models are a good indicator of the anti-caries effect the fluoride dentifrice will exert in humans.

With all of the supporting evidence presented above, it is believed that the present invention enhances the anti-caries effect of fluoride.

### Novelty of the Mechanism of Action

Preventive dentistry has typically not taken advantage of the anti-caries effect of reducing cariogenic bacteria to address caries, as the use of fluoride, and its protective properties against tooth demineralization and enhancing tooth remineralization, is still the most widely recommended anti-caries agent. Two reasons for this include the following: 1) there is a lack of antibacterial agents that are truly effective against cariogenic bacteria present in dental biofilms and 2) there is a lack of drug systems which can deliver the therapeutic amounts that are required for an antibacterial agent to be effective against cariogenic bacteria present in dental biofilms. Further to this discussion, it is not inherent that adding an antibacterial agent to an oral care composition containing fluoride will enhance the anti-caries effect of fluoride, in part due to chemical incompatibility that may occur between clinical relevant concentrations of the antibacterial agent and the fluoride ion source and also the fact that clinical relevant concentrations of the antibacterial may be not be completely effective in the kill of cariogenic bacteria. According to Featherstone, "reducing the cariogenic bacterial challenge and enhancing the effect of fluoride by the use of new sustained-delivery systems would have a major effect on dealing with caries as a disease." (2006 Featherstone) It is thought that the present invention is novel based on the lack of such anti-caries agents and also fulfills this need for an anti-caries agent that enhances the effects of fluoride. This belief of novelty is substantiated when comparing the present invention with previous efforts to combine therapeutic levels of the antibacterial agent, chlorhexidine, with fluoride.

Chlorhexidine (CHX) is a compound with potent antibacterial properties and it is well-known to those skilled in the art as highly effective against plaque and gingivitis. The purpose of antiplaque and antigingival agents is to reduce inflammation of the gingival tissue in order to impede the development of periodontal disease. A 0.12% (w/v) CHX mouthrinse has been clinically shown to have a statistically significant effect against plaque and gingivitis in clinical trials, and this concentration of CHX is used throughout the in. (2006 Gunsolley, 2006 Featherstone) Since CHX is such a reliable antibacterial agent used against plaque and gingivitis, attempts have also been made to combine therapeutic levels of CHX and fluoride ion sources into oral care compositions for use against caries. One example of such a prior attempt was the effort to combine clinically effective concentrations of chlorhexidine and sodium monofluorophosphate. Through these attempts, it was determined that CHX and sodium monofluorophosphate are not compatible with one another in "clinically relevant concentrations in vitro". (2006 Kolahi) CHX and sodium monofluorophosphate are thought to interact with each other to form "a chlorhexidine-monofluorophosphate salt of low solubility in water." (1988 Barkvoll)

Since the 1970s, investigations have also been made concerning the feasibility and effectiveness of combining clinically relevant concentrations of CHX and sodium fluoride for anti-caries use. Initial studies for CHX-Fluoride oral care compositions investigated the synergy between the two compounds to achieve combined effectiveness against both caries and gingivitis. (1978 Luoma, 1976 Emilson, 2003 Freitas) It has since been demonstrated that combinations of CHX with fluoride can "increase the cariostatic effect of topical fluoride" (1994 Ogaard), but there still remains debate in the art about whether the fluoride-chlorhexidine association is beneficial since the CHX-F combination is believed to reduce the concentration of CHX. (1994 Ogaard, 2003 Freitas) There is further investigation, however, into whether known therapeutic levels of CHX (such as 0.12%) are truly reduced with the use of these sodium fluoride/chlorhexidine oral care compositions. One study from 2003 measured the *in vitro* substantivity of CHX of a 0.12% (w/v) CHX and 0.05% (w/v) sodium fluoride (CHX-NaF) oral rinse. The substantivity of CHX is one of its positive properties, and refers to "its intrinsic ability to be retained by oral surfaces, and gradually released into oral fluids over many hours." (2003 Freitas) The study found that there was a significant decrease in the substantivity of CHX in the CHX-NaF solution compared with a solution containing 0.12% (w/v) chlorhexidine gluconate alone. Further to this observation, there was less CHX initially released from enamel when the CHX-NaF solution was applied compared with the chlorhexidine gluconate solution. There are two possible explanations for these observations, one is that fluoride and chlorhexidine compete to adsorb to the enamel surface. Another explanation is that the positively charged CHX reacts with fluoride, which reduces the concentration of CHX and subsequently inhibits the antibacterial properties of CHX-NaF. The final observations of this study were that the "significant decrease in the substantivity of chlorhexidine" observed *in vitro* made the researchers have "new concerns about its interaction with sodium fluoride" and question the "benefit of the association of these two drugs." (2003 Freitas) There are clinical studies that present conflicting results about whether or not there is an enhanced and significant anti-caries effect to be gained from using chlorhexidine/fluoride in a combination product over using a product with fluoride alone. The results vary based on a number of factors including the concentration of CHX and fluoride applied along with the specific embodiment of the composition (rinse, varnish, gel) that is administered. (2001 Whelton)

The examples above regarding the attempts and outcomes of combining CHX with fluoride ion sources are given to demonstrate that it is not obvious to combine antibacterial agents with any fluoride ion source to enhance the anti-caries effects of fluoride or the overall compositions. There are different physiochemical properties that exist among antibacterial agents that impart unique mechanisms of action which may or may not result in anti-caries effectiveness when these agents are combined with fluoride. For the present invention, stabilized chlorine dioxide is the antibacterial agent and it is possible that the differing properties of CHX and stabilized chlorine dioxide may be in part determined by how effective each agent is in enhancing the anti-caries effects of fluoride. For instance, CHX is a cation with several positive charges, while stabilized chlorine dioxide is primarily anionic. These electrochemical differences contribute to different mechanisms of action for the antibacterial activity of these agents. CHX "binds readily to negatively charged bacterial cell walls and can thereby disrupt membrane integrity." (2003 Freitas) Furthermore, CHX has been shown to have greater effectiveness against gram positive bacteria than gram negative bacteria. (2003 Freitas) Stabilized chlorine dioxide, on the other hand, oxidizes biomolecules and volatile sulfur compounds and "can neutralize bacterial proteolytic enzymes." (1994 Chapek) Also, unlike CHX, stabilized chlorine dioxide is known to be more effective against gram negative bacteria than gram positive bacteria. In terms of kill of cariogenic bacteria, CHX has proven to effectively kill the cariogenic *mutans streptococci* present in dental plaque biofilm, and stabilized chlorine dioxide has been shown to "suppress salivary levels of the cariogenic bacteria *S. mutans."* (2006 Featherstone, 2001 Grootveld) CHX has also been shown to be "much less effective in reducing lactobacilli in human mouths," while stabilized chlorine dioxide has been shown to reduce significant amounts of lactobacilli in human saliva. (2006 Featherstone, 2001 Grootveld) Perhaps it is this distinction of lactobacilli kill by stabilized chlorine dioxide that allows the antibacterial agent of the present invention to enhance the anti-caries effect of fluoride in a different way than CHX combined with fluoride, because stabilized chlorine dioxide is able to disrupt the biofilm and have kill of these two types of cariogenic bacteria. This may account for the results seen in the rat caries test where the present invention outperformed the USP reference standard fluoride dentifrice (Table 3a and Table 3b). Featherstone states that "an improved antibacterial that is effective against both MS [mutans streptococci] and LB [lactobacilli] and has a daily-dosage mechanism would be optimal" for antibacterial approach to caries treatment, and it is possible that the present invention embodies such properties to enhance fluoride in ways that a known antibacterial (CHX) does not. (2006 Featherstone)

### Stability of the Invention

An objective of the present invention is to teach compositions containing levels of the fluoride ion source, chlorine dioxide source, and pH range of the final composition that can be maintained, within the levels and ranges specified by the present invention, when the final composition is placed under:
1) Normal storage conditions of approximately 25 degrees Celsius (C) and 60% Relative Humidity for a storage period of one (1) year, preferably two (2) years, but most preferred for three (3) years, or
2) Accelerated conditions of approximately 40 degrees C and 75% Relative Humidity for a storage period of three (3) months, but preferably six (6) months

*Stability* is defined for the purposes of the present invention as the ability to maintain levels of the fluoride ion source, chlorine dioxide source, and pH within the levels specified by the present invention when the final composition is placed under normal storage conditions and accelerated conditions (described above). Stability is defined in this manner because it is believed that the specifications for the fluoride ion source, chlorine dioxide source and pH are in ranges that will provide safe and effective anti-caries properties of the present invention. It is believed that this expectation of stability for the present invention is achievable based on a stability study that was conducted on a specific embodiment of the present invention. The results of this stability study are detailed in Table 5 below.

**Table 5. Accelerated Stability of Two Stabilized Chlorine Dioxide Dentifrices**

| Results of 90 day Accelerated Stability Data | | | | | | | | | | | | | | | | | | | |
|---|---|---|---|---|---|---|---|---|---|---|---|---|---|---|---|---|---|---|---|
| | | Initial | | | | 15 Days | | | 30 Days | | | | 60 Days | | | | 90 Days | | |
| Formula ID | Sample | pH | % ClO₂ | F (ppm) | | pH | % ClO₂ | | pH | % ClO₂ | F (ppm) | | pH | % ClO2 | F (ppm) | | pH | % ClO2 | F (ppm) |
| *NaF*/*ClO₂ Experimental Dentifrice* | *W* | 6.91 | 0.11 | 1106 | | 6.97 | 0.10 | | 6.99 | 0.07 | 1062 | | 6.92 | 0.06 | 1050 | | 6.98 | 0.04 | 1021 |
| | | 6.88 | 0.12 | 1120 | | 6.96 | 0.10 | | 6.86 | 0.07 | 1084 | | 6.90 | 0.06 | 1066 | | 6.91 | 0.05 | 1047 |
| *NaF*/*ClO₂ Experimental* | *X* | 6.88 | 0.11 | 1123 | | 6.89 | 0.10 | | 6.89 | 0.10 | 1077 | | 6.88 | 0.06 | 1067 | | 6.98 | 0.04 | 1034 |
| *Dentifrice* | | 6.86 | 0.12 | 1110 | | 6.93 | 0.11 | | 6.93 | 0.10 | 1089 | | 6.87 | 0.06 | 1082 | | 6.91 | 0.04 | 1031 |
| *Stabilized* ClO₂ | *Y* | 7.92 | 0.11 | -- | | 7.98 | 0.08 | | 7.82 | 0.06 | -- | | 7.73 | 0.01 | -- | | 7.75 | 0.00 | -- |
| *Dentifrice* | | 7.87 | 0.13 | -- | | 7.98 | 0.09 | | 7.76 | 0.06 | -- | | 7.71 | 0.01 | -- | | 7.74 | 0.00 | -- |
| *Stabilized ClO₂* | *Z* | 7.86 | 0.12 | -- | | 7.88 | 0.10 | | 7.78 | 0.07 | -- | | 7.72 | 0.01 | -- | | 7.76 | 0.00 | -- |
| *Dentifrice* | | 7.86 | 0.11 | -- | | 7.88 | 0.10 | | 7.74 | 0.06 | -- | | 7.70 | 0.01 | -- | | 7.72 | 0.00 | -- |

| | | | | | | | | | | | | | | | | | | | |
|---|---|---|---|---|---|---|---|---|---|---|---|---|---|---|---|---|---|---|---|
| Table 5. Accelerated conditions were 40°C and 75% Relative Humidity. '--' indicates this attribute was not tested at this time point. | | | | | | | | | | | | | | | | | | | |

Two duplicate 7 oz samples of a single-phase dentifrice containing 0.24% (w/w) Sodium Fluoride and 0.125% (w/w) Stabilized Chlorine Dioxide (Sample W and Sample X) and two duplicate 7 oz samples of a single phase dentifrice containing 0.125% (w/w) Stabilized Chlorine Dioxide without fluoride (Sample Y and Sample Z) were placed in accelerated conditions for 90 days. Each sample was tested twice for the designated attributes (pH, ClO2 concentration and Fluoride Ion Concentration). The experimental NaF/ClO₂ dentifrice (Sample W and Sample X) retained between 0.04-0.05% or 400-500 ppm of stabilized chlorine dioxide (well within the levels of stabilized chlorine dioxide claimed in the present invention) compared with the stabilized chlorine dioxide dentifrice without sodium fluoride (Sample Y and Sample Z) which had no detectable stabilized chlorine dioxide remaining at the end of three months. Furthermore, the NaF/ClO₂ experimental dentifrice retained more than 90% of the initial fluoride ion at the end of three months, or 1021-1047 ppm of the fluoride ion, which is well within the fluoride ion levels taught by the present invention. It is expected that other embodiments of the present invention will be able to maintain similar stability based on these observations.

### Application of Composition and Various Uses

For the method of enhancing anti-caries efficacy, compositions of the present invention may be applied to the oral cavity, which includes but is not limited to the gingiva, tooth, and/or tongue. The compositions may be delivered to the oral cavity through various routes of topical administration. In one embodiment, the fluoride ion source and chlorine dioxide source of the oral compositions may be delivered to the oral cavity as a dentifrice, where the consumer would use a toothbrush to brush and apply the dentifrice to the teeth, gingiva, tongue, saliva and other relevant tissues in the oral cavity. A small, pea-size amount of the dentifrice could be applied with the toothbrush to the oral cavity, twice daily for 2 minutes, in the morning and at night before bed.

### EXAMPLES OF THE PRESENT INVENTION

The disclosed examples are only provided for additional instruction on possible embodiments of the present invention. The examples below are not intended to restrict or limit the scope of the present invention, as it is understood that there are various other embodiments that may be derived.

### Example 1

### Single-Phase Dentifrice

### Formulation

A formulation for a single phase dentifrice is disclosed below. The single phase dentifrice includes ingredients selected from the following components: Sodium Fluoride, Stabilized Chlorine Dioxide, Sodium Phosphate Monobasic, Sodium Phosphate Dibasic, Titanium Dioxide, Cellulose Gum, Hydrated Silica, Sorbitol, Sweetening Agent, Flavoring Agent, and Purified USP grade de-ionized Water.

### Ingredient Wt/Wt%

Sodium Fluoride 0.240%
Chlorine Dioxide (Stabilized 5% solution 2.500%
Sodium Phosphate Monobasic and Sodium Phosphate Dibasic % to achieve a Final pH of 6.7-7.0

The remaining excipient ingredients (e.g. Cellulose Gum, Hydrated Silica, Titanium Dioxide, Sorbitol, Sweetening Agent, Flavoring Agent, and De-Ionized Water) are to be selected and added in appropriate concentrations for dentifrice, which is known to those skilled in the art.

The pH of the final dentifrice composition (Example 1) is in the range of pH 6.7-7.0.

### Dentifrice Preparation Procedure

Aqueous soluble solid components (buffers and sweeteners) are dissolved in water which is added to Cellulose Gum. A mixture of water-insoluble components (e.g.titanium dioxide, hydrated silicas) is prepared and triturated with liquid polyols (e.g. sorbitol). The resultant paste is combined with the aqueous solution containing soluble excipients and chlorine dioxide solution using a homogenizer.

### Method for Application and Use of the Dentifrice

It is instructed that a quantity (approximate size of a pea) of the dentifrice composition be placed on a toothbrush. Teeth should be brushed with the dentifrice for a minimum of 1 minute and a frequency of two times a day, preferably in the morning and at night before bed. This method is instructed for individuals older than 6 years of age.

### Example 2

### Single-Phase Oral Rinse

A formulation for a single-phase oral rinse is disclosed below. The single phase oral rinse includes ingredients selected from the following components: Sodium Fluoride, Chlorine Dioxide, Citric Acid, Trisodium Phosphate, and Purified USP grade de-ionized water.

### Ingredient Wt/Wt%

Sodium Fluoride 0.050%
Chlorine Dioxide (Stabilized 5% solution) 2.500%
Citric Acid % to achieve a Final pH of 6.7-7.0

The remaining ingredients, e.g, trisodium phosphate and de-ionized water, are added in appropriate amounts to prevent the escape of chlorine dioxide and balance the overall formulation, which are known to those skilled in the art.

The final pH of the above oral rinse formulation is pH 6.7-7.0.

Humectants, sweetening agents, and flavoring agents may be added to the above oral rinse embodiment in concentrations known to those skilled in the art.

The sources of the ingredients are as follows: Sodium Fluoride (puriss. USP, Ph. Eur. grade powder, sold by Sigma-Aldrich), Chlorine Dioxide (Stabilized 5% solution, sold by Bio-Cide International), Trisodium Phosphate (ICL Performance Products), and Citric Acid (USP Anhydrous solution, sold by Jungbunzlauer).

### Oral Rinse Preparation Procedure

Trisodium phosphate and aqueous soluble active ingredients of the present invention (e.g. sodium fluoride and chlorine dioxide) are dissolved in de-ionized water. Citric acid is dissolved in de-ionized water in a separate preparation. Then the citric acid preparation is added and mixed into to the aqueous preparation (containing the sodium fluoride and chlorine dioxide) to achieve a pH of 6.7-7.0.

### Method for Application and Use of the Dentifrice

In another possible embodiment of the invention, the fluoride ion source and the chlorine dioxide source of the oral care composition may be delivered to the oral cavity as an oral rinse solution. It is instructed that the consumer would swish with 15 mL of the oral rinse for 30 seconds to 1 minute and expectorate the liquid once finished. The preferred frequency of administration would be two times a day (in the morning and at night before bed). This method is instructed for individuals older than 6 years of age.

### References

Aoba T, Fejerskov O. Dental Fluorsis: Chemistry and Biology. Crit Rev Oral Biol Med 2002;13(2):155-170.
Barkvoll P, Rolla G, Bellagamba S. Interaction between Chlorhexidine Digluconate and Sodium Monofluorophosphate in vitro. Scand JDent Res 1988;96(1):30-33.
Bouillaguet S. Biological Risks of Resin-Based Materials to the Dentin-Pulpal Complex. Crit Rev Oral Biol Med 2004; 15(1):47-60.
Braly A, et al. The Effect of Prism Orientation in the Indentation Testing of Human Molar Enamel. Arch Oral Biol 2007;52(9):856-860.
Chape CW, Reed OK, Ratcliff PA. Management of Periodontitis with Oral-Care Products. Compend Contin Educ Dent 1994;XV(6):740-746.
Cury JA, Tenuta LMA. Enamel remineralization: Controlling the Caries Disease or Treating Early Caries Lesions. Braz Oral Res 2009;23 Spec Issue 1:23-30.
Cury JA, Tenuta LMA. How to Maintain a Cariostatic Fluoride Concentration in the Oral Environment. Adv Dent Res 2008;20:13-16.
Denes G, Lazanas G. Oxidation of SnF2 Stannous Fluoride in Aqueous Solutions. Hyperfine Interact 1994;90:435-439.
Edgar WM, Higham SM. Role of Saliva in Caries Models. Adv Dent Res 1995;9(3);235-238.
Emilson CG, Krasse B, Westergren G. Effect of a Fluoride-Containing Chlorhexidine Gel on Bacteria in Human Plaque. Scand J Dent Res 1980;88:22-27.
European Commission: Enterprise Directorate-General, Pharmaceuticals and Cosmetics. Volume 1 Cosmetic Legislation - Cosmetic Products. 1999.
Featherstone JDB. Caries Prevention and Reversal Based on the Caries Balance. Pediatric Dentistry 2006:28(2); 128-132.
Featherstone JDB. Delivery Challenges for Fluoride, Chlorhexidine, and Xylitol. BMC Oral Health 2006;6:S8.
Food and Drug Administration. Anticaries Drug Products for Over-The-Counter Human Use; Final Monograph. 1995. USC Title 21 Parts 310, 355, 369.
Freitas CS, et al. Evaluation of the Substantivity of Chlorhexidine in Association with Sodium Fluoride In Vitro. Pesqui Odontol Bras 2003;17(1):78-81.
Garcia-Godoy F, Hicks J. Maintaining the Integrity of the Enamel Surface: The Role of Dental Biofilm, Saliva, and Preventive Agents in Enamel Demineralization and Remineralization. J Am Dent Assoc 2008;139:25S-34S.
Grootveld, M, et. al. Evidence for the Microbicidal Activity of a Chlorine Dioxide-Containing Oral Rinse Formulation In Vivo. J Clin Dent 2001;XII(3):67-69.
Gunsolley J. A Meta-Analysis of Six-Month Studies of Antiplaque and Antigingivitis Agents. JAm Dent Assoc 2006;137:1649-1657.
Harakeh S, Illescas A, Matin A. Inactivation of Bacteria by Purogene. JAppl Bacteriol 1988;64(5):459-463.
Kolahi J, Soolari A. Rinsing with Chlorhexidine Gluconate Solution After Brushing and Flossing Teeth: A Systematic Review of Effectiveness. Quintessence Int 2006;37(8):605-612.
International Conference on Harmonisation of Technical Requirements for Registration of Pharmaceuticals for Human Use (ICH). Guidance for Industry Q1A(R2) Stability Testing of New Drug Substances and Products. 2003.
Islam B, Khan SN, Khan Asad. Dental Caries: From Infection to Prevention. Med Sci Monit 2007;13(11):RA196-203.
Keyes PH. Dental Caries in the Molar Teeth of Rats. II. A Method for Diagnosing and Scoring Several Types of Lesions Simultaneously. J of Dent Res 1958;17(6):1088-1099.
Kidd EAM, Fejerskov O. What Constitutes Dental Caries? Histopathology of Carious Enamel and Dentin Related to the Action of Cariogenic Biofilms. JofDent Res 2004;83C:C35-C38.
Krishnaraju RK, Hart TC, Schleyer TK. Comparative Genomics and Structure Prediction of Dental Matrix Proteins. Adv Dent Res 2003;17:100-113.
Lendennman U, Grogan J, Oppenheim FG. Saliva and Dental Pellicle - A Review. Adv Dent Res 2000; 14:22-28.
Leone CW, Oppenheim FG. Physical and Chemical Aspects of Saliva as Indicators of Risk for Dental Caries in Humans. J Dent Educ 2001;65(10):1054-1059.
Luoma HH, et al. A Simultaneous Reduction of Caries and Gingivitis in a Group of Schoolchildren Receiving Chlorhexidine-Fluoride Applications. Results After 2 Years. Caries Res 1978;12:290-298.
Lynch E, et al. Multicomponent Spectroscopic Investigations of Salivary of Antioxidant Consumption by an Oral Rinse Preparation Containing the Stable Free Radical Species Chlorine Dioxide (ClO2•). Free Radic Res 1997;26(3):209-234.
Margolis HC, Beniash E, Fowler CE. Role of Macromolecular Assembly of Enamel Matrix Proteins in Enamel Formation. JofDent Res 2006;85(9):775-93.
Masschelein. Chlorine Dioxide - Chemistry and Environmental Impact of Oxychlorine Compounds. 1979. Ann Arbor Science Publishers, Inc., Ann Arbor, Michigan.
Mjor IA. Dentin Permeability: The Basis for Understanding Pulp Reactions and Adhesive Technology. Braz Dent J 2009;20(1):3-16*.*
Nguyen DH, Martin JT. Common Dental Infections in the Primary Care Setting. Am Fam Physician 2008;77(6);797-802.
Ogaard B, Seppa L, Rolla G. Professional Topical Fluoride Applications-Clinical Efficacy and Mechanism of Action. Adv Dent Res 1994;8(2):190-201.
Parshley DH. Dynamics of the Pulpo-Dentin Complex. Crit Rev Oral Biol Med 1996;7(2):104-133.
The Proprietary Association Subgroup on Fluoride Dentifrices. Standards for Fluoride Dentifrices. 1978. Robinson C, et al. The Chemistry of Enamel Caries. Crit Rev Oral Biol Med 2000;11(4):481-495.
Stookey GK, et al. Animal Caries Models for Evaluating Fluoride Dentifrices. Adv Dent Res 1995;9(3):198-207.
Villhauer A, Olson B, Drake D. Bactericidal Activity of Stabilized Chlorine Dioxide Against Polymicrobial Biofilms. 2009 International Association for Dental Research Poster #3417, General Session, Apr 1-4, 2009.
Wang L, et al. Mimicking the Self-Organized Microstructure of Tooth Enamel. JPhys Chem C Nanomater Interfaces 2008;112(15):5892-5899.
Warrick JM, et al. Caries-preventive Effects of Sodium and Amine Fluoride Dentifrices. Am J Dent 1999;12(1): 9-13.
Whelton H, O'Mullane D. The Use of Combination of Caries Preventive Procedures. J Dent Educ 2001;65(10):1110-1113.
Wirthlin MR, Marshall GW, Rowland RW. Formation ad Decontamination of Biofilms in Dental Unit Waterlines. J Periodontol 2001;74(11):1595-1609.
Yu D, et al. Caries Inhibition Efficacy of an Antiplaque/Antigingivitis Dentifrice. Am J Dent 2000;14:14C-17C.
Zero DT. Dentifrices, Mouthwashes, and Remineralization/Caries Arrestment Strategies. BMC Oral Health 2006;6(Suppl I):S9.

## Claims

1. A composition for treatment or prevention of caries, the composition comprising:
a) stabilized chlorine dioxide for reaction with acidic elements in the oral cavity to produce chlorine dioxide for the benefit of its bactericidal properties on an oral biofilm;
b) fluoride ion wherein said fluoride ion is from sodium fluoride, to reduce demineralization and promote remineralization of the teeth as a result of the bactericidal effect of the chlorine dioxide on the oral biofilm ; and
c) a buffer for establishing the effective pH range of the composition.

2. The composition as set forth in Claim 1 wherein said buffer is selected from a group consisting of acetate, citrate and phosphate buffers.

3. The composition as set forth in Claim 1 wherein said composition is in the form of a paste and the concentration of chlorine dioxide is in the range of about 0.005% to about 0.800% (w/w).

4. The composition as set forth in Claim 3 wherein concentration of the fluoride ion is in the range of about 45 ppm to about 5000 ppm.

5. The composition as set forth in Claim 3 wherein the buffer provides a pH in the range of about 6.0 to about 7.4.

6. The composition as set forth in Claim 1 or Claim 3 wherein said composition is a single phase composition.

7. A composition as defined in any of Claims 1 to 6 for use in a method for reducing demineralization of teeth, said method including the steps of:
a) applying a composition including stabilized chlorine dioxide for reaction with the acidic elements in the oral cavity to produce chlorine dioxide for the benefit of its bactericidal properties on an oral biofilm;
b) applying a fluoride ion as part of the composition to reduce demineralization and promote remineralization of the teeth resulting from the bactericidal effect of the chlorine dioxide on the oral biofilm; and
c) establishing the effective pH range of the composition with a buffer.

8. The composition for use as set forth in Claim 7 wherein said steps of applying is carried out by chlorine dioxide in a concentration range of about 0.005% to about 0.800% (w/w if the composition is not a liquid and w/v if the composition is not a solid).

9. The composition for use as set forth in Claim 7 or Claim 8 wherein said step of applying is carried out by the fluoride ion in the range of about 45 ppm to about 5000 ppm.

10. The composition for use as set forth in any one of Claims 7, 8 or 9 wherein said step of establishing is carried out by the buffer setting the pH range of the composition in the range of about 6.0 to about 7.4.

## Patentansprüche

1. Zusammensetzung zur Behandlung oder Vorbeugung von Karies, wobei die Zusammensetzung Folgendes aufweist:
a) stabilisiertes Chlordioxid für die Reaktion mit säurehaltigen Bestandteilen in der Mundhöhle, um Chlordioxid zu produzieren zugunsten von seinen bakteriziden Eigenschaften auf einen oralen Biofilm;
b) Fluoridionen, wobei die Fluoridionen aus Natriumfluorid stammen, um die Entmineralisierung zu verringern und die Remineralisierung der Zähne zu fördern, und zwar als Folge der bakteriziden Wirkung des Chlordioxids auf den oralen Biofilm; und
c) einen Puffer zum Herstellen des wirksamen pH-Bereiches der Zusammensetzung.

2. Zusammensetzung nach Anspruch 1, wobei der Puffer aus einer Gruppe ausgewählt ist, die aus Azetat-, Zitrat- und Phosphat-Puffern besteht.

3. Zusammensetzung nach Anspruch 1, wobei die Zusammensetzung in Form von einer Paste vorliegt und die Konzentration des Chlordioxids im Bereich von ungefähr 0,005% bis ungefähr 0,800% (w/w bzw. Gewichts-%) liegt.

4. Zusammensetzung nach Anspruch 3, wobei die Konzentration der Fluoridionen im Bereich von ungefähr 45 ppm bis ungefähr 5000 ppm liegt.

5. Zusammensetzung nach Anspruch 3, wobei der Puffer einen pH-Wert im Bereich von etwa 6,0 bis etwa 7,4 vorsieht.

6. Zusammensetzung nach Anspruch 1 oder Anspruch 3, wobei die Zusammensetzung eine einphasige Zusammensetzung ist.

7. Zusammensetzung, wie sie nach einem der Ansprüche 1 bis 6 definiert ist, zur Verwendung bei einem Verfahren zum Verringern der Entmineralisierung der Zähne, wobei das Verfahren folgende Schritte aufweist:
a) Auftragen einer Zusammensetzung, die stabilisiertes Chlordioxid zur Reaktion mit säurehaltigen Elementen in der Mundhöhle aufweist, um Chlordioxid zu produzieren zugunsten seiner bakteriziden Eigenschaften auf einen oralen Biofilm;
b) Auftragen von Fluoridionen, wobei die Fluoridionen von Natriumfluorid stammen, um die Entmineralisierung zu verringern und die Remineralisierung der Zähne zu fördern, und zwar als Folge der bakteriziden Wirkung des Chlordioxids auf den oralen Biofilm; und
c) Herstellen des wirksamen pH-Bereiches der Zusammensetzung mit einem Puffer.

8. Zusammensetzung zur Verwendung nach Anspruch 7, wobei der Schritt des Auftragens durch Chordioxid ausgeführt wird in einem Konzentrationsbereich von ungefähr 0,005% bis ungefähr 0,800% (w/w bzw. Gewichts-%, wenn die Zusammensetzung keine Flüssigkeit ist, und w/v bzw. Volumen-%, wenn die Zusammensetzung kein Feststoff ist).

9. Zusammensetzung zur Verwendung nach Anspruch 7 oder Anspruch 8, wobei der Schritt des Auftragens durch die Fluoridionen ausgeführt wird in einem Bereich von ungefähr 45 ppm bis ungefähr 5000 ppm.

10. Zusammensetzung zur Verwendung nach einem der Ansprüche 7, 8 oder 9, wobei der Schritt des Herstellens durch den Puffer ausgeführt wird, der den pH-Bereich der Zusammensetzung auf den Bereich von etwa 6,0 bis etwa 7,4 einstellt.

## Revendications

1. Composition pour le traitement ou la prévention des caries, la composition comprenant :
a) du dioxyde de chlore stabilisé pour réagir avec des éléments acides se trouvant dans la cavité buccale pour produire du dioxyde de chlore pour profiter de ses propriétés bactéricides sur un biofilm oral ;
b) des ions fluorure, les ions fluorure provenant de fluorure de sodium, pour réduire la déminéralisation et favoriser la re-minéralisation des dents, grâce à l'effet bactéricide du dioxyde de chlore sur le biofilm oral ; et
c) un tampon pour établir la plage effective de pH de la composition.

2. Composition selon la revendication 1, dans laquelle le tampon est sélectionné dans le groupe comprenant les tampons d'acétate, de citrate et de phosphate.

3. Composition selon la revendication 1, dans laquelle la composition a la forme d'une pâte et la concentration en dioxyde de chlore est comprise entre environ 0,005 % et environ 0,800 % (en poids).

4. Composition selon la revendication 3, dans laquelle la concentration en ions fluorure est comprise entre environ 45 ppm et environ 5000 ppm.

5. Composition selon la revendication 3, dans laquelle le tampon assure un pH compris entre environ 6,0 et environ 7,4.

6. Composition selon la revendication 1 ou la revendication 3, dans laquelle la composition est une composition à une seule phase.

7. Composition selon l'une quelconque des revendications 1 à 6, pour utilisation dans un procédé de réduction de la déminéralisation des dents, le procédé comprenant les étapes suivantes :
a) appliquer une composition comprenant du dioxyde de chlore stabilisé pour réagir avec les éléments acides se trouvant dans la cavité buccale pour produire du dioxyde de chlore pour profiter de ses propriétés bactéricides sur un biofilm oral ;
b) appliquer des ions fluorure, en tant que partie de la composition, pour réduire la déminéralisation et favoriser la re-minéralisation des dents grâce à l'effet bactéricide du dioxyde de chlore sur le biofilm oral ; et
c) établir la plage effective de pH de la composition à l'aide d'un tampon.

8. Composition pour une utilisation selon la revendication 7, dans laquelle les étapes d'application sont réalisées avec du dioxyde de chlore ayant une concentration comprise entre environ 0,005 % et environ 0,800 % (en poids si la composition n'est pas liquide, et en rapport poids/volume si la composition n'est pas solide).

9. Composition pour utilisation selon la revendication 7 ou la revendication 8, dans laquelle l'étape d'application est réalisée avec les ions chlorure présents dans une plage comprise entre environ 45 ppm et environ 5000 ppm.

10. Composition pour utilisation selon l'une quelconque des revendications 7, 8 ou 9, dans laquelle l'étape d'établissement est réalisée par le fait que le tampon fixe la plage de pH de la composition dans une plage comprise entre environ 6,0 et environ 7,4.
